(19) Europäisches Patentamt
European Patent Office
Office européen des brevets

(11) Veröffentlichungsnummer: **0 363 760 A1**

# (12) EUROPÄISCHE PATENTANMELDUNG

(21) Anmeldenummer: **89118114.1**

(51) Int. Cl.$^5$: **C07C 65/26**

(22) Anmeldetag: **29.09.89**

(30) Priorität: **12.10.88 DE 3834661**
**28.06.89 DE 3921165**

(43) Veröffentlichungstag der Anmeldung:
**18.04.90 Patentblatt 90/16**

(84) Benannte Vertragsstaaten:
**BE DE ES FR GB IT NL**

(71) Anmelder: **BAYER AG**

**D-5090 Leverkusen 1 Bayerwerk(DE)**

(72) Erfinder: **Reuter, Knud, Dr.**
**Scheiblerstrasse 99**
**D-4150 Krefeld(DE)**
Erfinder: **Freitag, Dieter, Dr.**
**Hasenheide 10**
**D-4150 Krefeld(DE)**
Erfinder: **Weymanns, Günther, Dr.**
**Karl-Arnold-Strasse 4**
**D-5090 Leverkusen 1(DE)**
Erfinder: **Dhein, Rolf, Dr.**
**Deswatinesstrasse 30**
**D-4150 Krefeld(DE)**
Erfinder: **Mayska, Paul Johannes, Dr.**
**Bodelschwinghstrasse 18**
**D-4150 Krefeld(DE)**
Erfinder: **Idel, Karsten-Josef, Dr.**
**Am Schwarzkamp 38**
**D-4150 Krefeld(DE)**
Erfinder: **Eckhardt, Volker, Dr.**
**Bodelschwinghstrasse 14**
**D-4150 Krefeld(DE)**
Erfinder: **Westeppe, Uwe, Dr.**
**Volgelskamp 72**
**D-4020 Mettmann(DE)**

## (54) Aromatische Ether.

(57) Die neuen aromatischen Ether der Formeln

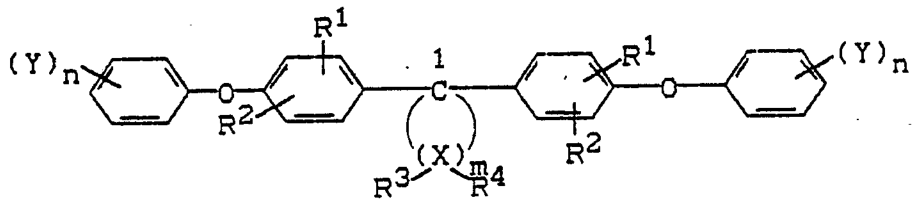

und

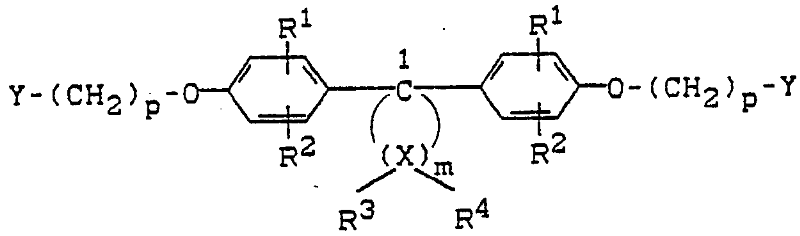

können verwendet werden zur Herstellung von Kunststoffen, die wiederum zu Formkörpern, Filmen, Folien und Filamenten weiterverarbeitet werden können. Die aus den neuen aromatischen Ethern hergestellten

EP 0 363 760 A1

Kunststoffe zeichnen sich aus durch eine besondere Wärmeformbeständigkeit.

## Aromatische Ether

Die Erfindung betrifft neue aromatische Ether, ein Verfahren zu ihrer Herstellung sowie die Verwendung der neuen aromatischen Ether zur Herstellung von Kunststoffen und Kunststoffmischungen, die zu Formkörpern, Filmen, Folien und Filamenten weiterverarbeitet werden können.

Gegenstand der Erfindung sind neue aromatische Ether der Formeln

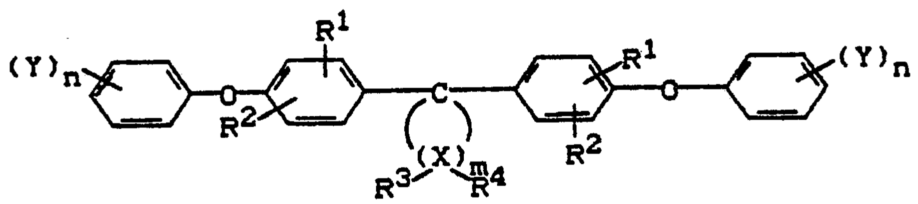
(I)

und

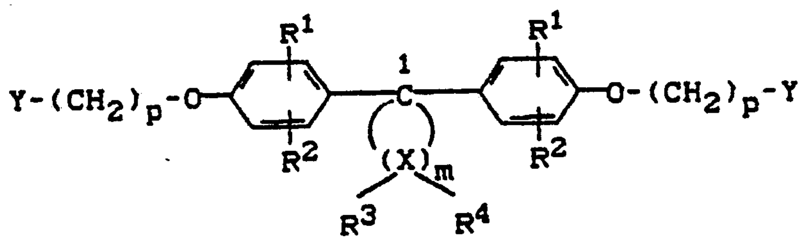
(II)

worin

$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl oder $C_7$-$C_{12}$-Aralkyl bedeuten,

$R^3$ und $R^4$ für jedes X individuell wählbar sind und unabhängig voneinander Wasserstoff oder $C_1$-$C_{12}$-Alkyl bedeuten,

X für Kohlenstoff steht mit der Maßgabe, daß mindestens ein Ringkohlenstoffatom gleichzeitig durch zwei $C_1$-$C_{12}$-Alkylreste substituiert ist,

Y für CN oder $COOR^5$ steht mit $R^5$ für H, $C_1$-$C_6$-Alkyl, $C_5$-$C_6$-Cycloalkyl oder $C_6$-$C_{10}$-Aryl,

n 1 oder 2 bedeutet,

m eine ganze Zahl von 4 bis 7 ist.

p für eine ganze Zahl von 1 bis 4 steht.

Als Halogene der oben angegebenen Formel kommen beispielsweise in Frage Fluor, Chlor und Brom, insbesondere Brom und Chlor, als Alkylreste der Methyl-, Ethyl-, n- und iso-Propyl- sowie der n-, iso- und tert.-Butylrest, bevorzugt der Methylrest, als Cycloalkylreste der Cyclopentyl- und der Cyclohexylrest, bevorzugt der Cyclohexylrest, als Arylreste der Phenyl und Naphthylrest, bevorzugt der Phenylrest, und als Aralkylreste der Benzyl- und der Cumylrest, bevorzugt der Cumylrest.

In der obengenannten Formel steht n bevorzugt für die Zahl 1 , m für die Zahlen 4 und 5, insbesondere für die Zahl 5 und p für Zahlen 1, 2 und 3, insbesondere für 1 und 2.

Bevorzugt sind 1 bis 2 Ringkohlenstoffatome (X), insbesondere nur 1 Ringkohlenstoffatom (X) der Formeln (I) und (II) gleichzeitig durch $R^3$ und $R^4$ substituiert, wobei die Substitution der Kohlenstoffatome in $\beta$-Stellung zu C-1 wiederum bevorzugt.

Bevorzugte aromatische Ether sind solche der Formeln

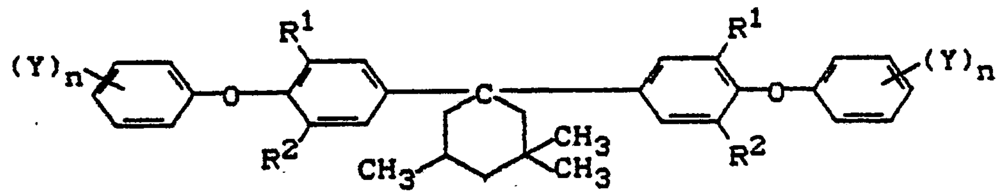

(III)

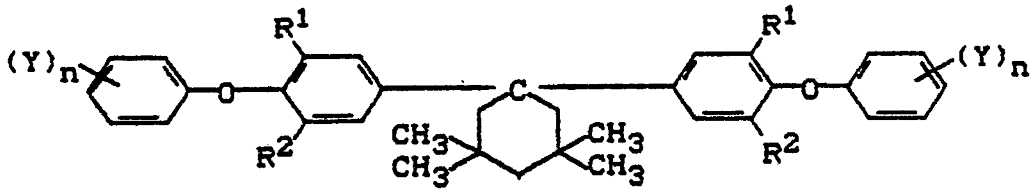

(IV)

und

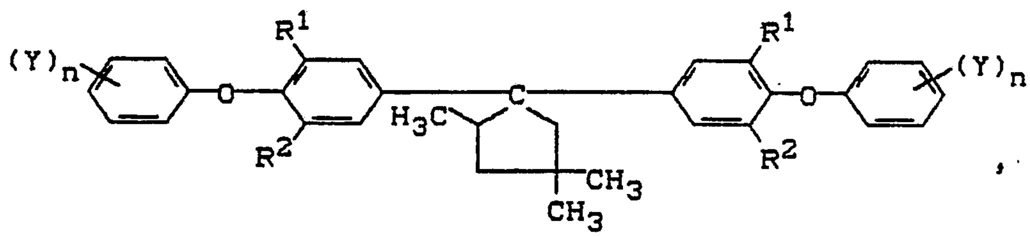

(V)

wobei

$R^1$, $R^2$, Y und n die für Formel (I) genannte Bedeutung besitzen.

Besonders bevorzugt sind aromatische Ether der Formel (II), in der die Reste $R^1$ und $R^2$ unabhängig voneinander Wasserstoff oder Methyl bedeuten und n für die Zahl 1 steht. Ganz besonders bevorzugt sind Verbindungen der Formel (II), in der $R^1$ und $R^2$ Wasserstoff bedeuten und n für die Zahl 1 steht.

Ein weiterer Gegenstand der vorliegenden Erfindung ist ein Verfahren zur Herstellung von aromatischen Ethern der Formeln (I) und (II)

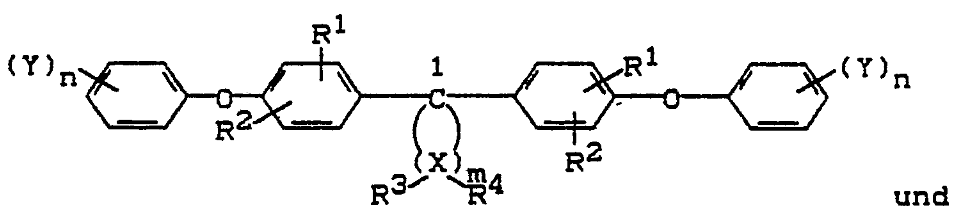

(I)

und

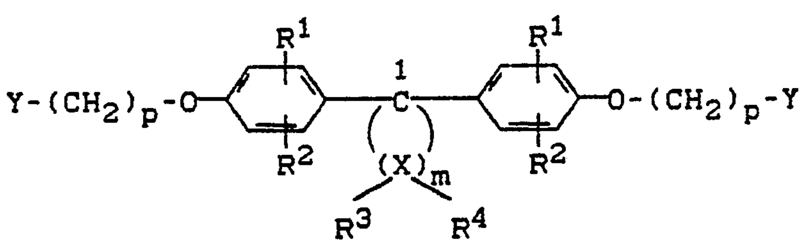

(II)

worin
$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl und $C_7$-$C_{12}$-Aralkyl bedeuten,
$R^3$ und $R^4$ für jedes X individuell wählbar sind und unabhängig voneinander Wasserstoff oder $C_1$-$C_{12}$-Alkyl bedeuten,
X für Kohlenstoff steht mit der Maßgabe, daß mindestens ein Ringkohlenstoffatom gleichzeitig durch zwei $C_1$-$C_{12}$-Alkylreste substituiert ist,
Y für CN oder $COOR^5$ steht mit $R^5$ für $C_1$-$C_6$-Alkyl, $C_5$-$C_6$-Cycloalkyl oder $C_6$-$C_{10}$-Aryl,
n 1 oder 2 bedeutet,
m eine ganze Zahl von 4 bis 7 ist,
p für eine ganze Zahl von 1 bis 4 steht,
das dadurch gekennzeichnet ist, daß man Verbindungen der Formel (VI)

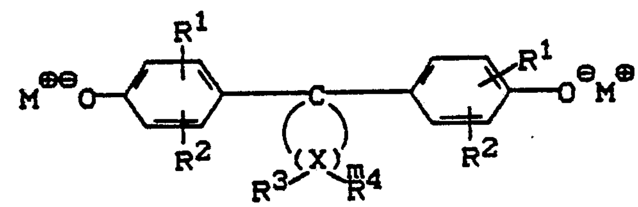

(VI) ,

worin
$R^1$, $R^2$, $R^3$, $R^4$, X und m die obengenannte Bedeutung haben und
M für ein Alkalimetall, insbesondere für Lithium, Natrium oder Kalium steht,
mit Verbindungen der Formel

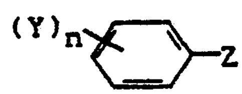

(VII)

oder

$$Y\text{-}(CH_2)_p\text{-}Z \quad \text{(VIII)},$$

worin
Y, n und p die obengenannte Bedeutung besitzen,
Z für Fluor, Chlor, Brom oder die Nitrogruppe, bevorzugt für Fluor, Chlor oder die Nitrogruppe steht,
bei Temperaturen von 20 bis 350 °C, bevorzugt 50 bis 250 °C, insbesondere 80 bis 100 °C, in Gegenwart eines dipolaren, aprotischen Lösungsmittels umsetzt.

Als dipolare, aprotische Lösungsmittel werden bevorzugt genannt: Acetonitril, Diethylenglykoldimethylether, N,N-Dimethylacetamid, N-Methylpyrrolidon, N-Methylcaprolactam, Diphenylsulfon, N,N-Dimethylformamid, N,N′-Dimethylimidazolidin-2-on(DMI) und/oder Dimethylsulfoxid, besonders bevorzugt Dimethylsulfoxid und DMI. Es ist auch möglich einen Teil, vorzugsweise bis zu ca. 50 Gew.-%, der dipolaren, aprotischen Lösungsmittel zu ersetzen durch unpolare Lösungsmittel, wie Toluol, Xylol, Mesitylen, Chlorbenzol, Cyclohexan und/oder Petrolether.

Die Menge des eingesetzten Lösungsmittels kann in weiten Bereichen schwanken. Im allgemeinen werden ca. 0,5 bis 50, bevorzugt 2 bis 20 Gew.-Teile Lösungsmittel, bezogen auf die Gesamtmenge an Verbindungen der Formel (V) und (VI), eingesetzt. Verwiesen wird in diesem Zusammenhang auf die US-PS 3.873.593, in der nähere Erläuterungen zu dem oben beschriebenen Verfahren zu entnehmen sind.

Die Verbindungen der Formel (VI) werden in Mengen von ca. 2 bis 3 Mol, bevorzugt 2 bis 2,5 Mol, bezogen auf 1 Mol der Verbindung der Formel (VII) eingesetzt.

Die eingesetzten Verbindungen der Formel (VII) sind bekannt und beispielsweise beschrieben in den US-Patentschriften 3.873.593, 3.763.210 und 3.787.475.

Die den Salzen der Verbindung (VI) zugrundeliegenden aromatischen Dihydroxyverbindungen können hergestellt werden durch Kondensation in an sich bekannter Weise der entsprechenden Phenole mit den entsprechenden Ketonen in Gegenwart von sauren Katalysatoren sowie gegebenenfalls weiteren Cokatalysatoren. Verwiesen wird in diesem Zusammenhang auf die deutsche Patentanmeldung P 38 32 3966 und auf Schnell, Chemistry and Physics of Polycarbonates, Interscience Publishers, New York, 1964.

Als in das erfindungsgemäße Verfahren einzusetzende Verbindungen der Formel (VI) kommen beispielsweise in Frage:

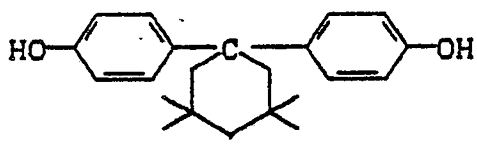 , 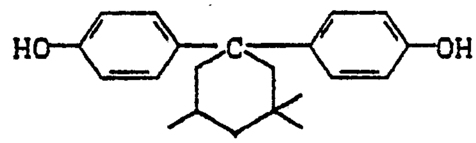 ,

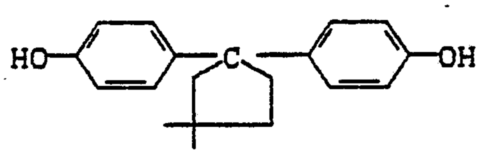 , 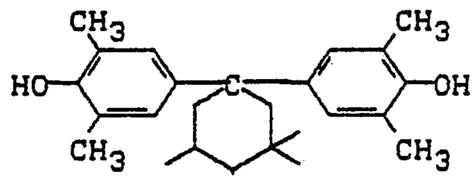 ,

besonders bevorzugt

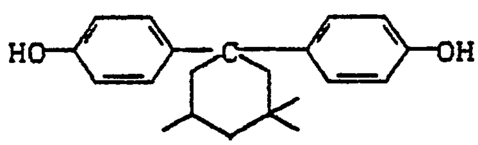 .

Als Verbindungen der Formel (VII) können z.B. eingesetzt werden:

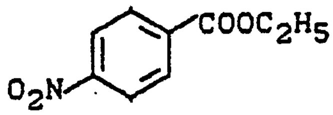 , 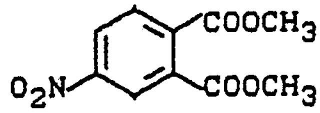 , 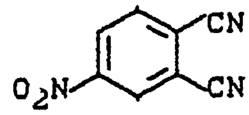 ,

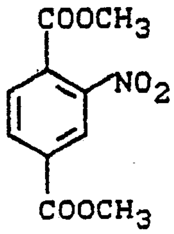 , 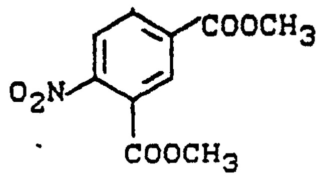 , 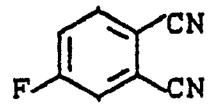 ,

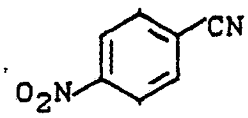 , 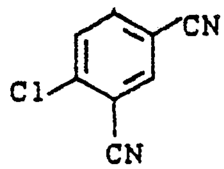 , 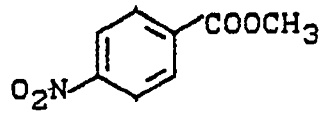 ,

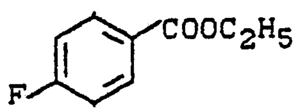 , 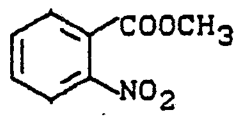 , 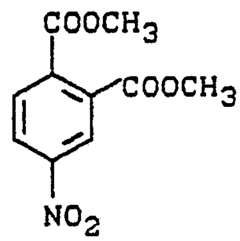 ,

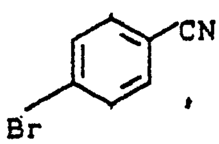 , 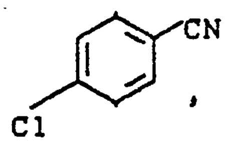 , $Br-CH_2COOC_2H_5$,

$Bv-CH_2-CN$, $Cl-CH_2COOH$,

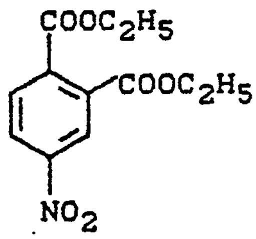,

bevorzugt

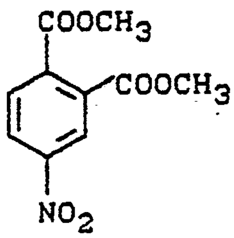, 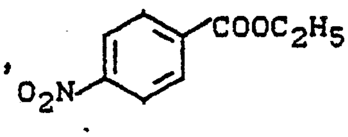, 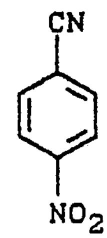, 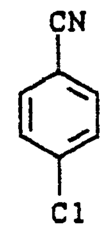

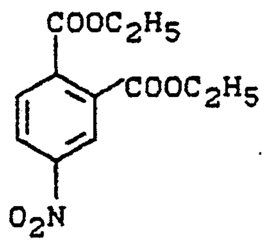, 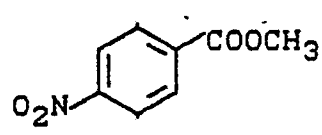,

besonders bevorzugt

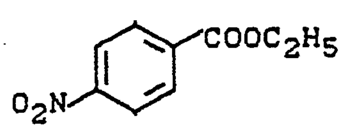, 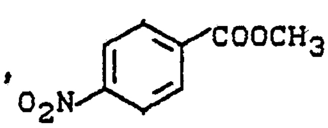, 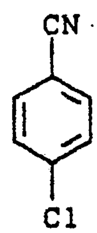, 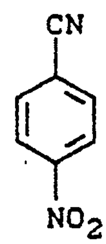.

Ein weiterer Gegenstand der vorliegenden Erfindung ist die Verwendung der neuen aromatischen Ether zur Herstellung von Polymeren, insbesondere von Duroplasten und Thermoplasten. Die aus den neuen aromatischen Ethern hergestellten Polymeren können wiederum verwendet werden für die Herstellung von Formkörpern, Filmen, Filamenten und Folien.

Beispielsweise können die neuen aromatischen Ether eingesetzt werden für die Herstellung von Polyetherimiden, aromatischen Polyestern, aliphatisch-aromatischen Polyestern, Polyamiden (aromatische und aliphatisch-aromatische), Duromeren durch Cyclotrimerisation der Nitrilgruppen, Polyetherketonen, bevorzugt für die Herstellung von Polyetherimiden, Polyestern und Polyamiden, besonders bevorzugt für die Herstellung von aromatischen und aromatisch-aliphatischen Polyestern sowie aromatischen und aromatisch-aliphatischen Polyamiden.

Zur Herstellung der Polymeren aus den neuen aromatischen Ethern werden die aromatischen Ether nach üblicher Weise mit den für die einzelnen Polymeren in Frage kommenden anderen Comonomeren polykondensiert. Die Herstellung der oben angeführten Polymeren kann dabei nach den literaturbekannten Verfahren beispielsweise über die Carbonsäureanhydride, die Carbonsäureester, die freien Carbonsäuren bzw. Amide (durch Verseifung der Ester bzw. Nitrile) oder die Säurechloride (siehe z.B. Houben-Weyl, Erweiterungs- und Folgebände zur 4. Auflage (1987), Band E 20, Teile 2, Seiten 1418-1429, 1456, 1522, 1524, 1527-1534, "Makromolekulare Stoffe") oder direkt aus den Verbindungen der Formeln (I) und (II) durchgeführt werden.

Selbstverständlich ist es auch möglich, die aus den neuen aromatischen Ethern hergestellten Polymeren untereinander oder mit anderen bekannten Polymeren, wie Polycarbonaten, Polyestercarbonaten, Polyestern, Polyimiden, Polyamiden, Polyetherimiden, Polyetherketonen, Polyethersulfonen und/oder aromatischen Polyethern, in üblicher Weise und in üblichen Mischungsverhältnissen abzumischen.

Die aus den neuen aromatischen Ethern hergestellten Polymeren besitzen gegenüber vergleichbaren Polymeren auf Basis von bekannten Dihydroxydiphenylcycloalkanen eine besonders gute Wärmeformbeständigkeit.

## Beispiele

### Beispiel 1

15,5 g des Bisphenols

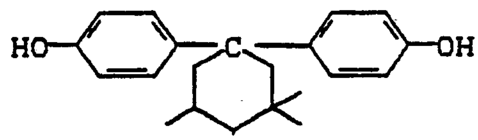

(0,05 M), 8,9 g 45 %ige Natronlauge (0,1 M), 100 ml Dimethylsulfoxid und 60 ml Toluol werden in einer Rührapparatur mit Wasserabscheider unter $N_2$ zum Rückfluß erhitzt, bis kein Wasser mehr abgeschieden wird. Danach wird statt Wasserabscheider eine Soxhlet-Apparatur aufgesetzt, die mit Molekularsieb 4 Å beschickt ist. Die Mischung wird 1 h unter $N_2$ zum Rückfluß erhitzt und anschließend die Soxhlet-Apparatur durch einen absteigenden Kühler ersetzt. Es wird so lange destilliert (in der Hauptsache Toluol), bis eine Innentemperatur von 145 °C erreicht ist. Danach werden 23,4 g p-Nitrobenzoesäureethylester zugegeben und das Reaktionsgemisch unter $N_2$ 12 h bei 120 °C umgesetzt. Nach Abkühlung wurde in viel $H_2O$ eingegossen und mehrfach mit Methylenchlorid ausgeschüttelt. Die Methylenchlorid-Phasen wurden eindestilliert. Der Rückstand (11 g) wurde durch NMR-Spektroskopie als in der Hauptsache

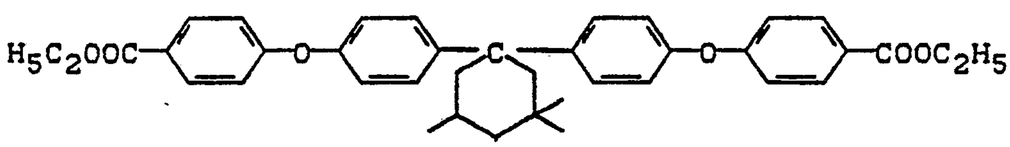

identifiziert.

### Beispiel 2

In einen 500 ml-Dreihalskolben, ausgerüstet mit Rührer, Thermometer, Wasserabscheider und Rückflußkühler werden 46,41 g (0,15 mol) Bis-(4-hydroxyphenyl)-3,3,5-trimethylcyclohexan, 42,08 g (0,30 mol) 40%ige Kalilauge, 80 g N,N′-Dimethylimidazolidin-2-on (DMI) und 100 ml Toluol chargiert und anschließend so lange am Rückfluß gehalten, bis alles Wasser aus dem System entfernt ist. Danach werden 41,25 g (0,30 mol) p-Chlorbenzonitril zugesetzt und die Reaktionsmischung auf 195 °C erhitzt. Nach 6 h Reaktionszeit wird die Reaktionsmischung am Rotationsverdampfer eingeengt und aus n-Butanol umkristallisiert.

Die Ausbeute des isolierten Produktes betrug 67 %. Das Produkt zeigte folgende Eigenschaften:
Schmelzpunkt: 175 °C
Gesamtchlor: 0,009 %
anorganisches Chlor: 16 ppm

| Elementaranalyse: | C | H | N | O |
|---|---|---|---|---|
| berechnet | 82,0 | 6,29 | 5,46 | 6,24 |
| gefunden | 82,2 | 6,60 | 5,67 | 6,40 |

Die $^1$H- und [13]-C-NMR-Spektren entsprechen der chemischen Formel:

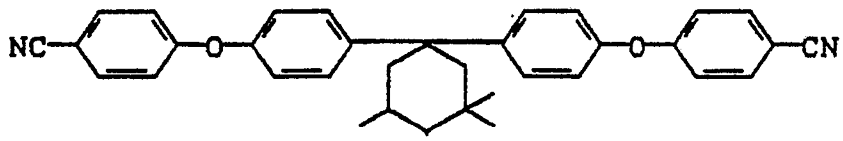

Beispiel 3

In einem 250-ml-Dreihalskolben, ausgerüstet mit Rührer, Thermometer, Gaseinleitungsrohr und Rückflußkühler werden 20,46 g (0,04 mol) 1,1-Bis[4-(cyanophenoxy)-phenyl]-3,3,5-trimethylcyclohexan (aus Beispiel 2), 44,8 g (0,4 mol) 50%ige Kalilauge und 50 ml DMI eingewogen. Die Reaktionsmischung wird unter Stickstoffeinleitung 24 h am Rückfluß gehalten. Während der Reaktion spaltet Ammoniak ab, und das anfangs kaum lösliche Nitril geht nach und nach in Lösung. Zum Schluß wird die Lösung klar. Das Fortschreiten der Reaktion wird durch Prüfung der Ammoniakentwicklung und durch Messung der Säurezahl des probeweise gefällten Produktes verfolgt. Nach beendeter Reaktion wird das Produkt durch Fällung in 600 ml verdünnter Salzsäure und anschließende Filtration isoliert.

Die Ausbeute war quantitativ. Das Produkt zeigte folgende Eigenschaften:
Schmelzpunkt: 273 °C (Essigsäure)
Säurezahl (SZ): 203 - 205 (theoretisch 204)

| Elementaranalyse: | C | H | O |
|---|---|---|---|
| berechnet | 76,34 | 6,22 | 17,43 |
| gefunden | 76,20 | 6,33 | 17,60 |

Die $^1$H- und $^{13}$C-NMR-Spektren bestätigen die chemische Formel:

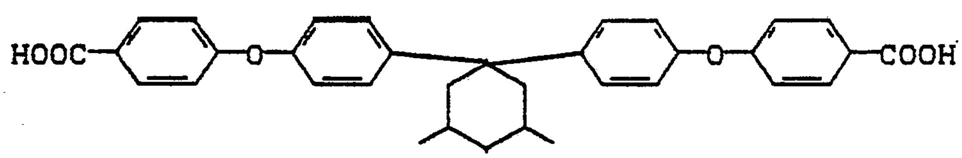

Beispiel 4

23,29 g des Bisphenols

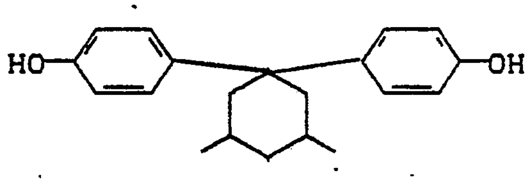

(0,075 M), 13,35 g 45 %ige Natronlauge (0,15 M), 100 ml Dimethylsulfoxid und 65 ml Toluol werden in

einer Rührapparatur mit Wasserabscheider unter $N_2$ zum Rückfluß erhitzt, bis kein Wasser mehr abgeschieden wird. Danach wird statt Wasserabscheider eine Soxhlet-Apparatur aufgesetzt, die mit Molekularsieb 4 Å beschicht ist. Die Mischung wird 1 h unter $N_2$ zum Rückfluß erhitzt und anschließend die Soxhlet-Apparatur durch einen absteigenden Kühler ersetzt. Es wird so lange destilliert (in der Hauptsache Toluol), bis eine Innentemperatur von 145 °C erreicht ist. Danach werden 24,44 g 4-Nitrobenzonitril (0,165 M) zugegeben und das Reaktionsgemisch unter $N_2$ 7 h bei 100 °C umgesetzt. Nach Abkühlen wurden die abgeschiedenen Kristalle abgesaugt, mit $H_2O$ und anschließend mit Methanol gewaschen und getrocknet.
Ausbeute: 24,92 g (65 % d.Th.)
Schmelzpunkt: 177-178 °C

| Elementaranalyse: | C | H | N |
|---|---|---|---|
| berechnet: | 82,0 | 6,29 | 5,46 |
| gefunden: | 81,7 | 6,55 | 5,66 |

Das 1H- und das IR-Spektrum entsprechen der chemischen Formel:

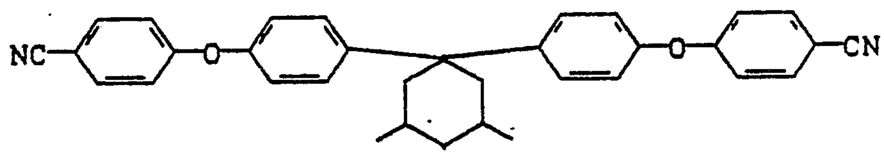

Beispiel 5

Analog Beispiel 1; jedoch mit einer Reaktionszeit von 13 h bei 150 °C, werden 23,3 g des Bisphenols

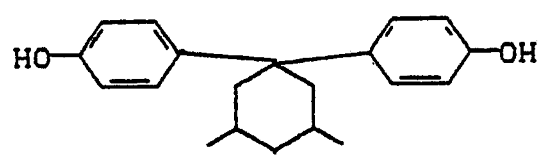

(0,075 M), 13,35 g 45 %ige Natronlauge (0,15 M), 100 ml Dimethylsulfoxid und 65 ml Toluol und 39,5 g Nitroterephthalsäure (0,165 M) umgesetzt. Nach Abkühlen wurde die Reaktionsmischung filtriert, mit $H_2O$ versetzt und an schließend mit Methylenchlorid extrahiert. Die $CH_2Cl_2$-Phase wurde eindestilliert, der Rückstand aus Toluol/Petrolether umgefällt (19 g Ausbeute) und als im wesentlichen

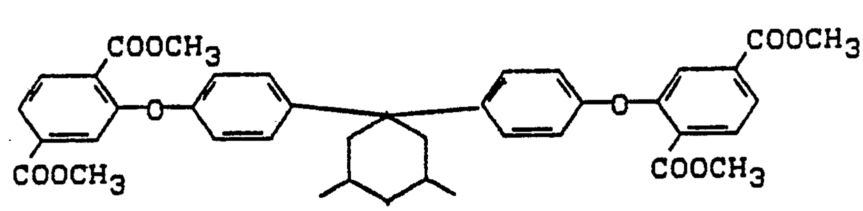

identifiziert.

Beispiel 6

60,8 g der Dicarbonsäure

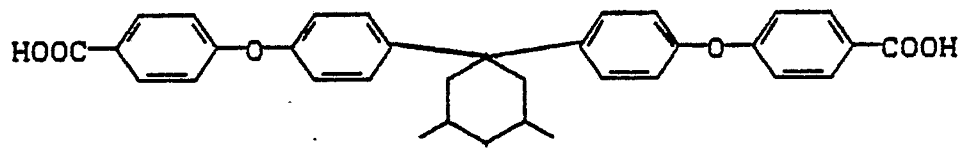

(hergestellt entsprechend Beispiel 3 oder durch Verseifung des Esters aus Beispiels 1) wurden in 130 g Thionylchlorid 15 h zum Rückfluß erhitzt. Danach wurde das überschüssige Thionylchlorid abdestilliert, der Rückstand in $CH_2Cl_2$ gelöst, gefiltert und $CH_2Cl_2$ wieder abdestilliert. Als Rückstand wurden 46,7 g des Säurechlorids

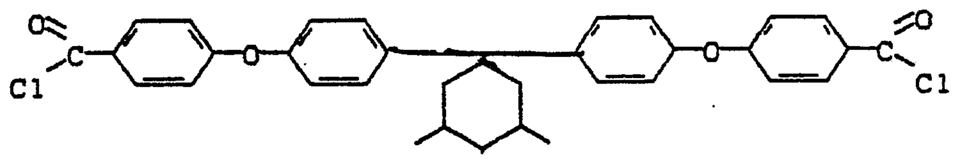

isoliert.

Beispiel 7

Zu einer Mischung von 10 g NaOH (0,25 M), 560 ml $H_2O$, 22,8 g Bisphenol A (0,1 M), 0,966 g Tetra-n-butylammonium-bromid und 450 ml $CH_2Cl$ wurde unter gutem Rühren bei 23 °C in 15 min. eine Lösung von 14,69 g (0,025 M) des Säurechlorids

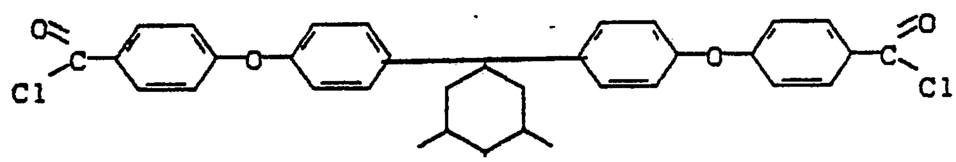

(aus Beispiel 6), 7,65 g Terephthaloylchlorid (0,0375 M) und 7,65 g Isophthaloylchlorid (0,0375 m) in 100 ml $CH_2Cl_2$ getropft. Danach wurde eine weitere Stunde bei 23 °C nachgerührt. Die $CH_2Cl_2$-Phase wurde anschließend mit $H_2O$ gewaschen. Aus der $CH_2Cl_2$-Phase wurden dann durch Abdampfen des Lösungsmittels 33,8 g eines amorphen, hochwärmeformbeständigen ($T_g$ = 189 °C) aromatischen Polyesters isoliert, aus dem zum Beispiel reißfeste Folien erhalten werden. Die relative Viskosität des Polyesters betrug $\eta_{rel}$ = 1,336 (5 g/l; $CH_2Cl_2$; 25 °C).

## Ansprüche

1. Aromatische Ether der Formeln

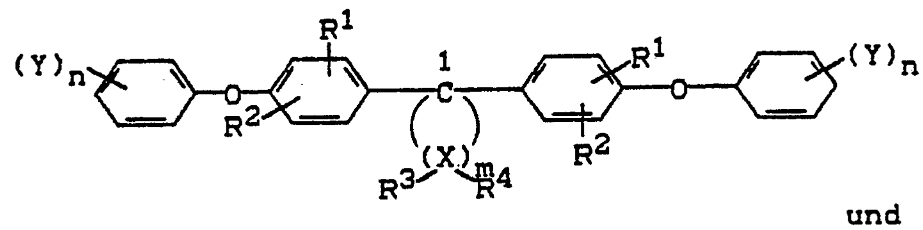

und

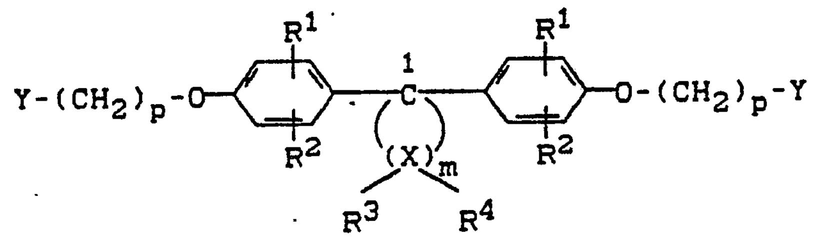

,

worin
$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl oder $C_7$-$C_{12}$-Aralkyl bedeuten,
$R^3$ und $R^4$ für jedes X individuell wählbar sind und unabhängig voneinander Wasserstoff oder $C_1$-$C_{12}$-Alkyl bedeuten,
X für Kohlenstoff steht mit der Maßgabe, daß mindestens ein Ringkohlenstoffatom gleichzeitig durch zwei $C_1$-$C_{12}$-Alkylreste substituiert ist,
Y für CN oder $COOR^5$ steht mit $R^5$ für H, $C_1$-$C_6$-Alkyl, $C_5$-$C_6$-Cycloalkyl oder $C_6$-$C_{10}$-Aryl,
n 1 oder 2 bedeutet,
m eine ganze Zahl von 4 bis 7 ist und
p für eine ganze Zahl von 1 bis 4 steht.

2. Verfahren zur Herstellung von aromatischen Ethern der Formeln

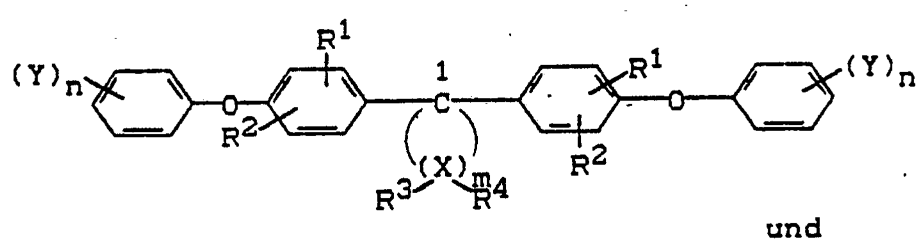

und

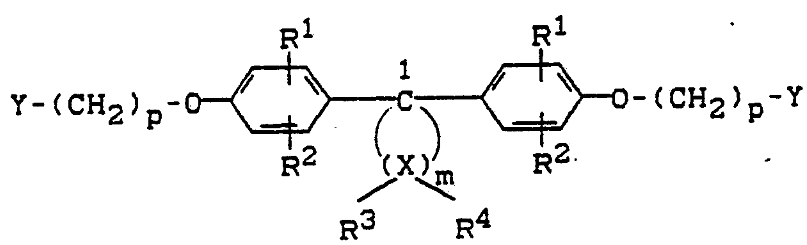

,

worin
$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl oder $C_7$-$C_{12}$-Aralkyl bedeuten,
$R^3$ und $R^4$ für jedes X individuell wählbar sind und unabhängig voneinander Wasserstoff oder $C_1$-$C_{12}$-Alkyl bedeuten,
X für Kohlenstoff steht mit der Maßgabe, daß mindestens ein Ringkohlenstoffatom gleichzeitig durch zwei $C_1$-$C_{12}$-Alkylreste substituiert ist,
Y für CN oder $COOR^5$ steht mit $R^5$ für $C_1$-$C_6$-Alkyl, $C_5$-$C_6$-Cycloalkyl oder $C_6$-$C_{10}$-Aryl,
n 1 oder 2 bedeutet,
m eine ganze Zahl von 4 bis 7 ist und

p für eine ganze Zahl von 1 bis 4 steht, dadurch gekennzeichnet, daß man Verbindungen der Formel

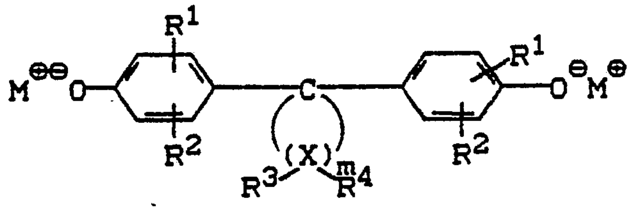

,

worin
$R^1$, $R^2$, $R^3$, $R^4$, X und m die obengenannte Bedeutung haben und
M für ein Alkalimetall steht,
mit Verbindungen der Formel

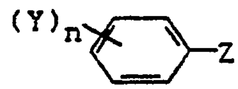

oder

$$Y-(CH_2)_p-Z,$$

worin
Y,n, und p die obengenannte Bedeutung besitzen und
Z für Fluor, Chlor, Brom oder die Nitrogruppe steht,
bei Temperaturen von 20 bis 350 °C in Gegenwart eines dipolaren, aprotischen Lösungsmittels umsetzt.

3. Verwendung der aromatischen Ether nach Anspruch 1 zur Herstellung von Kunststoffen und Kunststoffmischungen.

Europäisches Patentamt

# EUROPÄISCHER RECHERCHENBERICHT

Nummer der Anmeldung

EP 89 11 8114

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-3 944 583 (QUINN)<br>* Spalte 6, Zeilen 5-20 *<br>----- | 1 | C 07 C 65/26 |

| RECHERCHIERTE SACHGEBIETE (Int. Cl.5) |
|---|
| C 07 C 65/00 |

Der vorliegende Recherchenbericht wurde für alle Patentansprüche erstellt

| Recherchenort | Abschlußdatum der Recherche | Prüfer |
|---|---|---|
| DEN HAAG | 09-01-1990 | KLAG M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)